Europäisches Patentamt

(19) European Patent Office

Office européen des brevets

(11) Numéro de publication: **0 149 999**
**A2**

(12) # DEMANDE DE BREVET EUROPEEN

(21) Numéro de dépôt: **85100080.2**

(22) Date de dépôt: **04.01.85**

(51) Int. Cl.⁴: **A 41 B 13/02**

(30) Priorité: **06.01.84 FR 8400179**

(43) Date de publication de la demande:
**31.07.85 Bulletin 85/31**

(84) Etats contractants désignés:
**AT BE CH DE GB IT LI NL**

(71) Demandeur: **BOUSSAC SAINT FRERES B.S.F. Société anonyme dite:**
**12, rue du Vieux Faubourg**
**F-59800 Lille(FR)**

(72) Inventeur: **de Jonckheere, Raphael**
**797 Domaine de la Vigne**
**F-59910 Bondues(FR)**

(72) Inventeur: **Dussaud, Jacques**
**26 Avenue du Marechal Leclerc**
**F-59110 La Madelaine(FR)**

(74) Mandataire: **Casalonga, Alain et al,**
**BUREAU D.A. CASALONGA OFFICE JOSSE & PETIT**
**Baaderstrasse 12-14**
**D-8000 München 5(DE)**

(54) **Couche-culotte à étanchéité améliorée.**

(57) Couche-culotte comprenant un coussin absorbant (1) recouvert sur sa face inférieure d'une feuille souple (5) imperméable à l'humidité ayant une largeur et une longueur supérieures à celles du coussin (1), un voile (12) perméable à l'humidité recouvrant la face supérieure du coussin (1) et de la feuille (5), des moyens élastiques (9,111) étant fixé sur ladite feuille (5), le long des deux bords latéraux opposés (3) du coussin, et des moyens d'attache (13) étant prévus pour fermer la couche-culotte. La feuille (5) comporte deux rabats latéraux opposés (7) repliés autour des bords latéraux (3) du coussin (1) sur la face supérieure du coussin, les moyens élastiques (9,111) sont fixés à l'état tendu sur lesdits rabats (7), et le voile (12) recouvre la face supérieure des rabats (7) repliés et est fixé à cette face des rabats.

FIG.1

EP 0 149 999 A2

ment tendus lorsque la couche-culotte est de façon usuelle pliée sur elle-même en vue de son emballage.

La présente invention a pour objet une couche-culotte du type défini ci-dessus permettant d'améliorer l'effet d'étanchéité obtenu par des moyens élastiques dans la zone d'entre-jambes. L'invention a également pour objet une couche-culotte à étanchéité améliorée dans la zone d'entre-jambes, indépendamment des tolérances de positionnement du coussin absorbant par rapport à la feuille imperméable. L'invention a par ailleurs pour objet une couche-culotte à étanchéité améliorée indépendamment de l'épaisseur du coussin absorbant. L'invention a de plus pour objet une couche-culotte à étanchéité améliorée remédiant aux risques de formation de poches remplies d'humidité o de liquide susceptible d'entrer en contact avec la peau du porteur. L'invention a encore pour objet une couche-culotte à étanchéité améliorée réduisant le contact de la peau du porteur avec le coussin absorbant. L'invention a également pour objet une couche-culotte à étanchéité améliorée, permettant une fabrication simple à un prix de revient réduit. Enfin, l'invention a pou objet une couche-culotte sur laquelle les moyens élastiques se trouvent détendus lorsque la couche-culotte est pliée.

La couche-culotte conforme à l'invention comprend un coussin absorbant de forme générale rectangulaire, recouvert sur sa face inférieure par une feuille souple, imperméable à l'humidité, de largeur supérieure à celle du coussin. Cette feuille souple comporte deux rabats latéraux opposés repliés autour des bords latéraux du coussin absorbant sur la face supérieure du coussin absorbant, sur toute la longueur des bords latéraux de ce dernier. Des moyens élastiques sont fixés à l'état tendu sur lesdits rabats, le long des bords latéraux du coussin. Le voile perméable à l'humidité recouvre la face supérieure des rabats repliés et y est fixé.

La fixation des moyens élastiques sur les rabats latéraux de la feuill imperméable fait que lesdits moyens élastiques se trouvent toujours placés dans une position optimale par rapport à la peau du porteur, quelles que soient les tolérances d'épaisseur du coussin et/ou les tolérances de positi du coussin par rapport aux bords de la feuille imperméable dans la zone d'entre-jambes. Les rabats de la feuille imperméable sur la face supérieure du coussin font que la peau de l'utilisateur ne se trouve en contact qu'ave une partie de la face supérieure du coussin absorbant. Cela est certes déjà

Couche-culotte à étanchéité améliorée.

La présente invention se rapporte à une couche-culotte, notamment pour enfants en bas âge, comprenant un coussin absorbant de forme générale sensiblement rectangulaire, recouvert sur sa face inférieure d'une feuille souple imperméable à l'humidité ayant une largeur et une longueur supérieures à celles du coussin de manière à dépasser ce dernier latéralement et longitudinalement, ledit coussin et ladite feuille étant recouverts sur leur face supérieure par un voile perméable à l'humidité, des moyens élastiques étant fixés sur ladite feuille, le long des deux bords latéraux opposés du coussin et des moyens de fermeture étant prévus sur ladite feuille pour fermer la couche-culotte à hauteur de la taille du porteur.

De telles couches-culottes à jeter après usage, appelées également changes-complets, équipées de moyens élastiques dans la partie d'entre-jambes en vue d'améliorer l'étanchéité à cet endroit sont connues par exemple par les demandes de brevets français n° 2 063 794 et 2 251 278. Sur ces couches-culottes connues, les moyens élastiques sont fixés à la feuille imperméable à distance vers l'extérieur des bords latéraux du coussin absorbant, dans la zone d'entre-jambes. Si les moyens élastiques sont très éloignés des bords latéraux du coussin, il se forme, entre les moyens élastiques et les bords du coussin, des poches non garnies de matériau absorbant dans lesquelles de l'humidité, voire du liquide, peut s'accumuler et se trouve alors en contact avec la peau. Si, par contre, les moyens élastiques sont très proches des bords latéraux du coussin absorbant, les moyens élastiques risquent, selon l'épaisseur du coussin absorbant, de ne pas entrer correctement en contact avec la peau du porteur, ce qui donne lieu à des fuites. Ces deux problèmes peuvent d'ailleurs se poser sur une même couche-culotte si le coussin absorbant n'est pas placé exactement en position médiane par rapport aux moyens élastiques opposés.

On a également déjà proposé (demande de brevet français n° 2 501 972) de fixer les moyens élastiques sur la feuille imperméable de manière qu'ils se trouvent, dans la zone d'entre-jambes, à distance vers l'intérieur des bords latéraux du coussin absorbant. Les moyens élastiques serrent ainsi le coussin absorbant entre eux-mêmes et la peau de l'utilisateur, ce qui compromet l'effet d'étanchéité recherché. De plus, les moyens élastiques sont forte- connu en soi par exemple dans le brevet U.S. n° 3 386 442 et la demande de brevet français n° 2 285 820, mais ces documents ne suggèrent en aucune manière la prévision des moyens élastiques sur les rabats de la feuille imperméable.

Lesdits rabats de la feuille imperméable peuvent présenter une largeur constante telle que les extrémités libres des deux rabats opposés se trouvent à distance l'une de l'autre sur toute la longueur. Il est cependant possible également de donner auxdits rabats une plus grande largeur dans la zone des deux extrémités longitudinales et une plus faible largeur dans la zone intermédiaire, c'est-à-dire dans la zone d'entre-jambes, de telle manière que les deux rabats opposés se recouvrent dans la zone des deux extrémités longitudinales et laissent subsister entre eux une fenêtre dans la zone intermédiaire.

Les moyens élastiques peuvent être fixés sur les rabats de manière à se trouver, dans la zone d'entre-jambes, à l'extérieur des bords latéraux du coussin absorbant, à cheval sur ces bords latéraux ou de préférence à l'intérieur de ces bords latéraux du coussin absorbant. Notamment dans ce dernier cas, les moyens élastiques sont situés sur la partie du coussin qui, à l'état plié de la couche-culotte, se trouve en compression par rapport à la ligne neutre de coussin plié, de sorte que les moyens élastiques sont alors détendus.

Les moyens élastiques peuvent par ailleurs être fixés soit sur la face inférieure des rabats c'est-à-dire la face tournée vers le coussin absorbant, soit, de préférence, sur la face supérieure des rabats repliés. Dans ce dernier cas, il n'existe pas, entre les moyens élastiques et la peau du porteur, de couche de feuille imperméable qui, en raison des plis qu'elle forme sous l'effet de la contraction des moyens élastiques, pourrait éventuellement donner lieu à des fuites.

Le voile perméable à l'humidité qui est fixé par collage sur la face supérieure de la couche-culotte, sauf de préférence aux endroits où le coussin absorbant n'est pas recouvert par les rabats, assure l'immobilisation des rabats de la feuille imperméable sur la face supérieure du coussin absorbant. Les rabats ne sont alors avantageusement pas fixés sur la face supérieure du coussin, ce qui donne aux moyens élastiques une certaine liberté par rapport au coussin. Il en résulte une meilleure étanchéité et une détente complète des moyens élastiques à l'état plié de la couche-culotte.

Suivant un mode de réalisation avantageux de la couche-culotte conforme à l'invention, une échancrure est découpée, après repliage des rabats, sensiblement au milieu de la longueur de la couche-culotte c'est-à-dire dans la zone d'entre-jambes, dans les deux épaisseurs de la feuille imperméable, à partir de chaque bord latéral correspondant à la ligne de pliage du rabat. Afin d'assurer l'étanchéité de la couche-culotte dans la zone de ces échancrures découpées, les deux épaisseurs de feuille sont reliées entre elles par une ligne de collage ou de soudure le long du bord de ces échancrures.

En se référant aux dessins annexés, on va décrire ci-après plus en détail plusieurs modes de réalisation illustratifs et non limitatifs d'une couche-culotte conforme à l'invention; sur le dessin :

la figure 1 est une vue en plan, avec arrachement partiel, d'un premier mode de réalisation d'une couche-culotte conforme à l'invention;

la figure 2 est une coupe à plus grande échelle suivant II-II de la figures 1;

la figure 3 est une vue en plan, avec arrachement partiel, d'un second mode de réalisation d'une couche-culotte conforme à l'invention;

la figure 4 est une coupe à plus grande échelle suivant IV-IV de la figure 3.

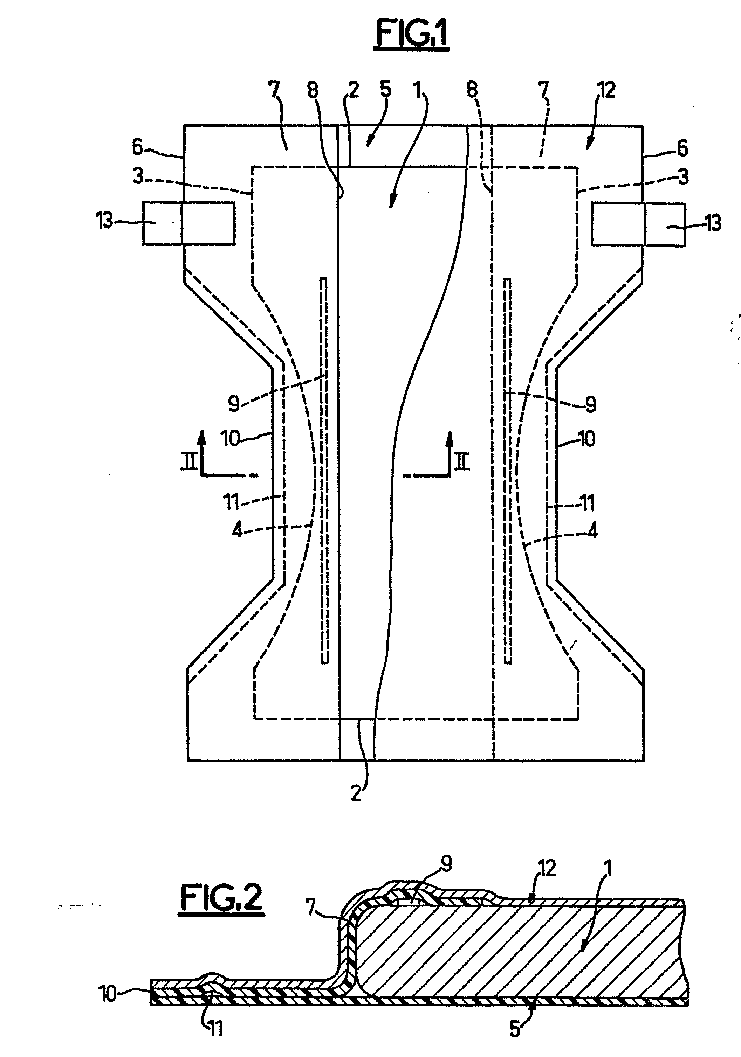

La couche-culotte illustrée par la figure 1 comprend un coussin absorbant 1 de forme générale rectangulaire. Les bords transversaux 2 du coussin correspondant aux petits côtés du rectangle sont rectilignes, tandis que les bords latéraux 3 correspondant aux grands côtés du rectangle présentent, sensiblement au milieu de leur longueur, chacun une découpe ou échancrure 4 en arc de cercle.

Le coussin 1 est fixé de façon usuelle, par exemple par des lignes longitudinales de colle, non représentées, sur une feuille 5 souple, imperméable à l'humidité, de formule générale rectangulaire. La feuille 5 présente, dans le sens longitudinal du coussin 1, une dimension (longueur) quelque peu supérieure à la longueur du coussin 1. Dans le sens transversal du coussin 1, la feuille 5 présente une dimension (largeur) nettement supérieure à la largeur du coussin 1. Ce dernier est placé en position médiane sur la feuille 5 de manière que la feuille 5 déborde de tous les côtés sur le coussin 1.

La largeur de la feuille 5 est choisie de manière que chacune des deux parties latérales de la feuille 5 s'étendant au-delà des bords latéraux 3 du coussin 1 puisse être repliée autour d'une ligne longitudinale 6 située à l'extérieur du bord latéral 3 correspondant du coussin 1, sous la forme d'un rabat 7 sur la face supérieure du coussin 1. On reconnaît sur les figures 1 et 2 que chaque rabat 7 recouvre le bord latéral 3 correspondant au coussin 1 sur toute la longueur de ce dernier, donc également à la hauteur des échancrure 4 du coussin. La largeur des rabats 7 est telle qu'une zone de largeur constante du coussin 1 reste dégagée, c'est-à-dire non recouverte, sur toute la longueur du coussin, entre les bords libres rectilignes 8 des rabats 7.

Chaque rabat 7 comporte un élément élastique 9 s'étendant le long du bord libre 8 du rabat 7, sur une partie au moins de la longueur dudit bord. Dans l'exemple représenté, l'élément élastique 9 s'étend sensiblement sur la longueur de l'échancrure 4 du coussin 1. L'élément élastique 9 est fixé à l'état tendu sur la face du rabat 7 qui vient en regard du coussin 1 lorsque le rabat 7 est replié sur le coussin 1. L'élément élastique 9 longe le bord 8 à faible distance de manière que sur toute sa longueur, il se trouve entre le bord latéral 3 du coussin 1 et le bord 8 du rabat 7, même à l'endroit de la partie la plus profonde de l'échancrure 4. L'élément élastique 9 est ainsi situé sur le dessus du coussin 1.

Une échancrure 10 est pratiquée dans le bord latéral 6 de la feuille 5, après repliage du rabat 7 avec l'élément élastique 9 sur le dessus du coussin 1. Cette échancrure 10 qui intéresse les deux épaisseurs de la feuille 5 est pratiquée à la hauteur de la découpe 4 du coussin 1 à une profondeur telle qu'elle pénètre en partie dans la découpe 4. Elle présente ici une forme trapézoïdale. Il serait toutefois possible également de donner à l'échancrure 10 une forme autre, par exemple en arc de cercle. L'échancrure 10 est longée sur toute sa longueur par une ligne de collage ou de soudage 11 rejoignant les deux parties du bord 6 situées de part et d'autre de l'échancrure 10. Cette ligne de collage ou de soudage 11 relie de façon étanche, sur toute la longueur de l'échancrure 10, le rabat 7 au pli inférieur de la feuille imperméable 5.

L'ensemble ainsi constitué (feuille imperméable 5 avec coussin absorbant 1 et rabats 7 avec éléments élastiques 9 repliés sur le dessus du coussin 1 et échancrés en 10) est recouvert sur toute sa face de dessus d'un voile 12 perméable à l'humidité. Le voile 12 est fixé au moins à sa périphérie, par collage ou soudage, aux rabats 7 et au pli inférieur de la feuille 5 à l'endroit où ce pli n'est pas recouvert par les rabats 7.

Enfin, deux éléments d'attache 13 par adhésif, de type connu en soi, servant à la fermeture de la couche-culotte autour de la taille du porteur, sont fixés à cheval sur la partie des bords latéraux 6 située d'un côté des échancrures 10.

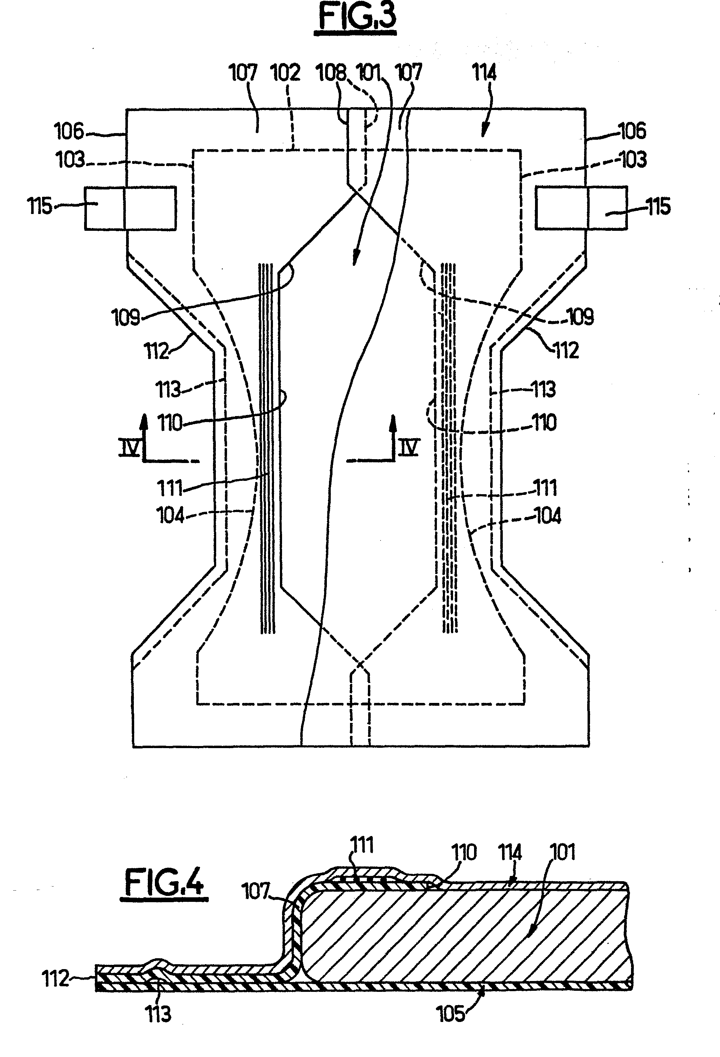

Les figures 3 et 4 représentent une variante d'une couche-culotte conforme à l'invention, qui diffère du mode de réalisation des figures 1 et 2 par la largeur de la feuille imperméable avant repliage des rabats, par la forme de ces rabats et par la nature et la disposition des éléments élastiques.

Un coussin absorbant 101 ayant la même forme que le coussin 1 des figures 1 et 2, c'est-à-dire une forme générale rectangulaire avec deux découpes 104 en arc de cercle dans ses deux bords latéraux opposés 103, est appliqué en position médiane et fixé par exemple par collage sur une feuille imperméable 105 de forme générale rectangulaire. La feuille 105 présente une longueur légèrement supérieure à la longueur du coussin 101 de sorte que les deux bords transversaux 102 du coussin 101 se trouvent en retrait par rapport aux bords correspondants de la feuille 105.

La feuille 105 présente, perpendiculairement à la longueur du coussin 101, une largeur supérieure à deux fois la largeur de la couche-culotte à fabriquer. Les deux parties latérales de la feuille 105 dépassant les bords latéraux 103 du coussin 101 sont repliées autour d'une ligne 106 située à distance à l'extérieur des bords 103, sous la forme de rabats 107 sur le dessus du coussin 101. La distance entre les lignes de pliage 106 des deux rabats 107, définissant la largeur de la couche-culotte, est légèrement inférieure à la moitié de la largeur de la feuille 105 perpendiculairement à la longueur du coussin 101. Par conséquent, les rabats 107 se recouvrent l'un l'autre à leur extrémité libre.

Chaque rabat 107 comporte, dans son bord libre 108, au milieu de la longueur dudit bord, sur une longueur inférieure à la longueur du coussin 101, une échancrure trapézoïdale 109. La profondeur des échancrures 109 est telle que les deux rabats 107 qui se recouvrent l'un l'autre par leurs extrémités de part et d'autre des échancrures 109 ménagent entre elles une ouverture ou fenêtre à l'endroit des échancrures 109 et que le fond 110 de chaque échancrure 109 se trouve situé à distance vers l'intérieur par rapport au fond de la découpe correspondante 104 du coussin 101. En d'autres termes, la largeur de la fenêtre ainsi formée qui laisse le coussin 101 dégagé, c'est-à-dire non recouvert, est inférieure à la larguer du coussin 101 à l'endroit des découpes 104, la longueur de la fenêtre étant inférieure à la longueur du coussin 101.

Chaque rabat 107 porte, sur sa partie située entre le fond 110 de l'échancrure 109 et le fond de la découpe 104 du coussin 101, un élément élastique 111 longitudinal. Cet élément élastique 111 est constitué ici par un élément multibrin formé de quatre minces brins élastiques fixés à l'état tendu par collage parallèlement côte à côte sur le rabat 107. Au lieu d'être fixé, comme les éléments élastiques 9 du mode de réalisation des figures 1 et 2, sur la face inférieure des rabats repliés, c'est-à-dire sur la face tournée vers le coussin 101, les éléments élastiques 111 sont fixés sur la face supérieure des rabats repliés 107, c'est-à-dire sur la face opposée au coussin 101.

On retrouve par ailleurs, dans le mode de réalisation suivant les figures 3 et 4, des échancrures 112 dans les bords latéraux 106 de la feuille imperméable 101, comparables aux échancrures 10 du mode de réalisation des figures 1 et 2, ainsi qu'une ligne de collage ou de soudage 113 longeant l'échancrure 112 pour relier entre eux, de façon étanche, le long de l'échancrure 112, le rabat 107 et le pli inférieur de la feuille imperméable 105.

On retrouve également, dans le mode de réalisation des figures 3 et 4, un voile 114 perméable à l'humidité, recouvrant, sur la face supérieure, l'ensemble formé par le coussin absorbant 101 et la feuille imperméable 105 dont les rabats 107 portant les éléments élastiques 111 sont rabattus sur le dessus du coussin 101. La couche-culotte comporte également deux éléments d'attache 115 par adhésif pour fermer la couche-culotte autour de la taille du porteur.

Dans les deux modes de réalisation représentés et décrits, les rabats 7, 107 sont avantageusement fixés par collage au pli inférieur de la feuille imperméable 5, 105 en dehors de la zone recouverte par le coussin absorbant 1, 101. Les rabats 107 peuvent, par ailleurs, être avantageusement fixés par collage l'un à l'autre dans les deux zones de recouvrement, dans le mode de réalisation des figures 3 et 4.

Par contre, il est préférable que les rabats 7, 107 ne soient pas fixés au coussin absorbant 1, 101 au moins au voisinage des éléments élastiques 9, 111, afin de donner à ces derniers une certaine liberté par rapport au coussin absorbant.

Il y a lieu de noter que les deux modes de réalisation décrits ci-dessus et illustrés par les dessins annexés n'ont été donnés qu'à titre indicatif et non limitatif et que de nombreuses modifications et variantes sont possibles dans le cadre de l'invention. Ainsi, les couches-culottes conformes à l'invention ne comportent pas obligatoirement des échancrures latérales 10, 112 dans la zone d'entre-jambes. Par ailleurs, les éléments élastiques multibrins 111 du mode de réalisation des figures 3 et 4 pourraient être remplacés par des éléments élastiques 9 en forme de ruban du mode de réalisation des figures 1 et 2, et inversement. Les éléments élastiques 9 du mode de réalisation des figures 1 et 2 pourraient également être rapportés sur la face supérieure des rabats 7, comme les éléments élastiques 111 du mode de réalisation des figures 3 et 4, et inversement. De la même manière, les deux formes de rabats 7 et 107 peuvent être utilisés indifféremment en combinaison avec des éléments élastiques multibrins ou en forme de ruban, appliqués sur la face supérieure ou sur la face inférieure des rabats.

Enfin, il y a lieu de noter que la nature des éléments constitutifs de la couche-culotte conforme à l'invention ne fait pas l'objet de la présente invention et qu'il est possible de faire appel à des matériaux usuels pour la fabrication de couches-culottes, par exemple à de la cellulose du type "fluff", éventuellement entourée d'une enveloppe de non-tissé ou de papier, pour le coussin absorbant 1, 101, à une matière plastique telle que le polyéthylène pour la feuille imperméable 5, 105, à un non-tissé hydrophobe, par exemple de polypropylène, pour le voile 12, 114 perméable à l'humidité.

REVENDICATIONS

1.Couche-culotte comprenant un coussin absorbant (1,101) de forme générale sensiblement rectangulaire, recouvert sur sa face inférieure d'une feuille souple (5,105) imperméable à l'humidité ayant une largeur et une longueur supérieures à celles du coussin (1,101) de manière à dépasser ce dernier latéralement et longitudinalement, un voile (12, 114) perméable à l'humidité recouvrant la face supérieure du coussin (1, 101) et de la feuille (5, 105), des moyens élastiques (9,111) étant fixés sur ladite feuille (5,105), le long des deux bords latéraux opposés (3,103) du coussin, et des moyens d'attache (13,115) étant prévus sur ladite feuille pour fermer la couche-culotte à hauteur de la taille du porteur, caractérisée par le fait que la feuille imperméable (5,105) comporte deux rabats latéraux opposés (7,107) repliés autour des bords latéraux (3,103) du coussin absorbant (1,101) sur la face supérieure du coussin absorbant, sur toute la longueur desdits bords latéraux, que les moyens élastiques (9,111) sont fixés à l'état tendu sur lesdits rabats (7,107), et que le voile (12, 114) recouvre la face supérieure des rabats (7, 107) repliés et est fixé à cette face des rabats.

2. Couche-culotte suivant la revendication1, caractérisée par le fait que les moyens élastiques (9,111) sont fixés sur les rabats (7,107) à distance vers l'intérieur par rapport aux bords latéraux (3,4; 103, 104) du coussin absorbant (1,101) dans la zone d'entre-jambes.

3. Couche-culotte suivant la revendication 1 ou 2, caractérisée par le fait que les moyens élastiques (9,111) sont fixés sur la face des rabats (7,107) repliés, tournée vers le coussin absorbant (1,101).

4. Couche-culotte suivant la revendication 1 ou 2, caractérisée par le fait que les moyens élastiques (9,111) sont fixés sur la face des rabats (7,107) repliés, opposée au coussin (1,101).

5. Couche-culotte suivant la revendication 4 ou 5, caractérisée par le fait que lesdits moyens élastiques sont constitués par un élastique (9) en forme de ruban.

6. Couche-culotte suivant la revendication 3 ou 4, caractérisée par le fait que lesdits moyens élastiques sont constitués par un élastique multibrin (111).

7. Couche-culotte suivant l'une quelconque des revendications précédentes, caractérisée par le fait que la feuille imperméable (105) présente une largeur supérieure à deux fois la largeur de la couche-culotte, et comporte une échancrure (109) dans chacun de ses bords latéraux (108), au milieu de la longueur desdits bords, sur une longueur inférieure à la longueur du coussin absorbant (101), de sorte que les deux rabats (107) repliés se recouvrent dans la zone des deux extrémités longitudinales et laissent subsister entre eux une fenêtre dans la zone intermédiaire.

8. Couche-culotte suivant l'une quelconque des revendications précédentes, caractérisée par le fait qu'elle comporte une échancrure (10, 112) dans chaque bord latéral (6,106) et que les deux plis de la feuille imperméable (5, 105) sont reliés entre eux par une ligne de collage ou soudage (11, 113) sur toute la longueur desdites échancrures.

9. Couche-culotte suivant l'une quelconque des revendications précédentes, caractérisée par le fait que les rabats (7,107) sont fixés par collage sur le pli inférieur de la feuille imperméable (5,105) autour du coussin absorbant (1,101).

10. Couche-culotte suivant la revendication 7, caractérisée par le fait que les rabats (7,107) sont fixés par collage sur le pli inférieur de la feuille imperméable (105) autour du coussin (101) et l'un à l'autre dans leurs zones de recouvrement.

FIG.1

FIG.2

## FIG.3

## FIG.4